# EUROPÄISCHE PATENTSCHRIFT

(11) **EP 3 718 464 B1**
(45) Veröffentlichungstag und Bekanntmachung des Hinweises auf die Patenterteilung: **07.06.2023**
(21) Anmeldenummer: 20167634.3
(22) Anmeldetag: 01.04.2020
(51) Int. Cl.: A61B 1/00, A61B 1/05, A61B 1/06, A61B 1/07, G02B 23/24

(54) **ENDOSKOPISCHES INSTRUMENT**
ENDOSCOPIC INSTRUMENT
INSTRUMENT ENDOSCOPIQUE

(30) Priorität: 03.04.2019 DE 102019204759
(43) Veröffentlichungstag der Anmeldung: 07.10.2020
(73) Patentinhaber: Richard Wolf GmbH, 75438 Knittlingen (DE)
(72) Erfinder: Weber, Bernd Claus, 76228 Karlsruhe (DE); Dolt, Martin, 75438 Knittlingen (DE); Tewes, Moritz, 75015 Bretten (DE); Rennert, Jens, 76137 Karlsruhe (DE)
(74) Vertreter: Patentanwälte Vollmann Hemmer Lindfeld Partnerschaft mbB

(56) Entgegenhaltungen:
- WO-A1-2004/032731
- US-A1- 2008 062 429
- US-A1- 2008 130 108
- US-A1- 2014 204 188
- US-A1- 2015 141 753

## Beschreibung

Die vorliegende Offenbarung betrifft ein endoskopisches Instrument für medizinische oder technische Zwecke mit einem distalen länglichen Einführabschnitt zum Einführen in einen zu untersuchenden Hohlraum in einem organischen Körper oder einem technischen Gerät.

Es ist bekannt, Endoskope dazu zu nutzen, um Videoaufnahmen vom Inneren eines menschlichen oder tierischen Körpers zu Zwecken der medizinischen Diagnose und/oder Therapie zu machen. Außerdem werden Endoskope bei der Untersuchung von technischen Geräten eingesetzt. Dabei ist es ein ständiges Bestreben, den Einführabschnitt von Endoskopen möglichst dünn auszugestalten, damit möglichst kleine Hohlräume eingesehen werden können und das Gewebe oder technische Gerät dabei möglichst wenig in Mitleidenschaft zu ziehen.

Das Innere von menschlichen und tierischen Hohlorganen ist von einer Schutzschicht ausgekleidet, der sogenannten Schleimhaut (Mukosa). Die Schleimhaut ist von besonderem medizinischen Interesse, da sie sich in exponierter Lage gegenüber äußeren Einflüssen, insbesondere gegenüber gewebeverändernden und -schädigenden Einflüssen befindet, z.B. im Mund, Rachen, Kehlkopf, Bronchien oder Lunge etwaigem Rauch oder Giftstoffen in der Atemluft ausgesetzt ist, und deshalb in vielen Fällen Ausgangspunkt für pathologische Veränderungen des Organs ist. Außerdem ist die Schleimhaut vergleichsweise einfach endoskopisch und damit minimalinvasiv und deshalb patientenschonend diagnostizierbar und gegebenenfalls auch therapierbar.

Problematisch für die endoskopische Untersuchung der Schleimhaut ist allerdings der Schleim, den die Schleimhaut als schützende Flüssigkeit auf ihrer Oberfläche im Hohlraum anreichert. Der Schleim ist zwar transparent, jedoch bildet er einen glatten Film, der Lichteinfall reflektiert. Da bei einem Endoskop der Bildgebungspfad und der Beleuchtungspfad oft eng zusammen liegen, beeinträchtigen die Reflexionen an der Schleimoberfläche oft deutlich das Bild von der Schleimhaut selbst. Das gleiche Problem tritt bei der endoskopischen Untersuchung von technischen Geräten auf, wenn glatte, beispielsweise metallische oder spiegelnde, Flächen das Beleuchtungslicht aus dem Beleuchtungspfad direkt in den Bildgebungspfad reflektieren.

Die WO 2010/014072 A1 versucht dieses Problem dadurch zu lösen, dass im Bildgebungspfad sowie im Beleuchtungspfad jeweils dichroitische Linearpolarisatoren angeordnet sind, deren Polarisationsebenen über Kreuz liegen, sodass reflektiertes Licht ausgefiltert wird und im Wesentlichen nur gestreutes Licht auf einen bildgebenden Sensor im Bildgebungspfad trifft. Dabei wird das physikalische Prinzip ausgenutzt, dass die Reflexion von polarisiertem Licht polarisationserhalten ist, wogegen die Streuung polarisationsmischend wirkt. Da im Gegensatz zum Schleim die Schleimhaut selbst das Beleuchtungslicht hauptsächlich streut und fast nicht reflektiert, kann mittels gekreuzter Linearpolarisatoren ein reflexreduziertes Bild von der Schleimhaut selbst erzielt werden.

Nachteilig an der in der WO 2010/014072 A1 beschriebenen Lösung ist allerdings, dass die Lichtausbeute mit weniger als 25% sehr gering ist und proximalseitig eine relativ hohe Lichtmenge in den Beleuchtungspfad eingekoppelt werden muss, damit für die Bildgebung genug Licht auf einen bildgebenden Sensor im Bildgebungspfad trifft. Ein großer Teil dieser hohen Menge eingekoppelten Lichts wird nämlich in den dichroitischen Linearpolarisatoren absorbiert und in Wärme umgewandelt. Dies ist nicht nur energetisch ineffizient, sondern führt zu einer Aufheizung des Endoskops. Im Hinblick auf eine etwaige Wärmedenaturierung von Körperproteinen muss sichergestellt sein, dass sich das Endoskop an keiner mit dem Körper in Kontakt stehenden Stelle über eine maximale Temperatur erhitzt. Außerdem kann ein dichroitischer Polarisator, der oft aus einer Folie mit organischem Material wie Polyvinylalkohol-Folie (PVA) oder Zellulosehydrat besteht, durch die Wärmeentwicklung selbst Schaden nehmen. Die US 2015/0141753 A1 beschreibt eine Bildverarbeitungseinrichtung, bei der ein Objekt mittels einer Beleuchtungseinheit abwechselnd mit einem ersten und einem zweiten Lichtstrahl beleuchtet wird, wobei die Lichtstrahlen unterschiedliche Polarisationsrichtungen haben.

Dokument US 2014/0204188 offenbart ein endoskopisches Instrument, welches am distalen Ende eine LED, eine Gitterstruktur und eine Linse aufweist.

Die vorliegende Offenbarung stellt dagegen ein endoskopisches Instrument vor, das zum einen effizient Reflexionen bei der Bildgebung reduziert und ggf. sogar minimiert, wobei gleichzeitig die Lichtausbeute verbessert ist und die Erwärmung von Komponenten im Beleuchtungspfad durch das Beleuchtungslicht geringer ausfällt. Außerdem wird eine Erhöhung der Farbsättigung und damit eine qualitative Verbesserung des endoskopischen Bildes erzielt.

Gemäß der vorliegenden Offenbarung wird ein endoskopisches Instrument mit einem distalen länglichen Einführabschnitt zum Einführen in einen Hohlraum bereitgestellt, wobei der Einführabschnitt mindestens einen Beleuchtungspfad und einen Bildgebungspfad aufweist, wobei der erste Beleuchtungspfad an einem distalen Ende des Einführabschnitts eine Lichtquelle in Form einer LED aufweist. Der mindestens eine Beleuchtungspfad weist dabei distalwärtig von der Lichtquelle eine Metallgitterstruktur auf, die einen ersten Anteil des Lichts der Lichtquelle mit einer bestimmten linearen Polarisation zumindest teilweise distalwärts transmittiert und einen zweiten Anteil des Lichts der Lichtquelle ohne diese Polarisation zumindest teilweise proximalwärts reflektiert, und der mindestens eine Beleuchtungspfad ist dabei distalwärtig von der Metallgitterstruktur linsenfrei.

Die Begriffe "distalwärtig" bzw. "proximalwärtig" sollen hierin eine relative Position bedeuten, die sich distal bzw. proximal von einer Bezugsposition befindet. Die Begriffe "distalseitig" bzw. "proximalseitig" sollen hierin eine Position an einer distalen bzw. proximalen Seite eines Gegenstands bedeuten. Die Begriffe "distalwärts" bzw. "proximalwärts" soll hierin eine Richtung bedeuten, die sich nach distal bzw. proximal erstreckt.

Das endoskopische Instrument weist vorzugsweise genau einen Bildgebungspfad auf, kann aber auch mehrere Bildgebungspfade aufweisen, beispielsweise für eine dreidimensionale Bildgebung. Das endoskopische Instrument weist vorzugsweise mehr als einen Beleuchtungspfad auf, kann aber auch nur genau einen Beleuchtungspfad aufweisen, beispielsweise wenn damit ausschließlich eine reflexreduzierte Bildgebung mit erhöhter Farbsättigung stattfinden soll.

Der von der Metallgitterstruktur distalwärts transmittierte erste Anteil des Lichts ist linear polarisiert. Die Metallgitterstruktur wirkt also hier wie ein Polarisator, der Lichtanteile ohne diese Polarisation allerdings nicht wie ein dichroitischer Polarisator absorbiert, sondern proximalwärts reflektiert. Dadurch heizt sich die Metallgitterstruktur nicht so sehr auf wie ein dichroitischer Polarisator. Außerdem kann der proximalwärts reflektierte zweite Anteil des Lichts, der ohne den polarisierten ersten Anteil selbst eine dazu komplementäre Polarisation hat, im Beleuchtungspfad dadurch zumindest teilweise "recycelt" werden, dass die komplementäre Polarisation wieder gemischt wird und an einer zumindest teilweise spiegelnden Fläche wieder distalwärts zurück auf die Metallgitterstruktur gelenkt wird. Die Lichtausbeute von polarisiertem Licht, das die Metallgitterstruktur letztlich distalwärts transmittiert, kann damit erhöht werden, ohne die erzeugte Lichtmenge bzw. Wärmemenge zu erhöhen. Zur Effizienz trägt zudem bei, dass der Beleuchtungspfad dabei distalwärtig von der Metallgitterstruktur linsenfrei ist. Dadurch wird der Polarisationsgrad des von der Metallgitterstruktur transmittierten Lichtanteils nicht wieder durch die Lichtstrahlformung einer Linse beeinträchtigt.

Das linear polarisierte Beleuchtungslicht trifft während der endoskopischen Untersuchung auf die Schleimhaut und den darauf befindlichen Schleim. Die Reflexion an der Schleimoberfläche ist polarisationserhaltend, während die Streuung an der Schleimhaut polarisationsmischend ist. Mit einem linear polarisierenden Medium im Bildgebungspfad kann beispielsweise bei entsprechend gekreuzter Ausrichtung der Polarisationsebene bezüglich der Metallgitterstruktur die Reflexion absorbiert, distalwärts reflektiert und/oder aus dem Bildgebungspfad weggebrochen werden, sodass hauptsächlich von der Schleimhaut gestreutes Licht auf einen bildgebenden Sensor im Bildgebungspfad trifft. Dabei wird zusätzlich eine erhöhte Farbsättigung des Bildes erreicht.

Erfindungsgemäß weist die Lichtquelle proximalseitig eine reflektierende Schicht auf, die zumindest teilweise den von der Metallgitterstruktur proximalwärts reflektierten zweiten Lichtanteil distalwärts reflektiert. Da die Lichtquelle eine LED ist, kann der Dioden-Chip bereits proximalseitig eine reflektierende Metallschicht aufweisen, die dazu dienen kann, den zweiten von der Metallgitterstruktur proximalwärts reflektierten Lichtanteil wieder distalwärts zu reflektieren.

Erfindungsgemäß weist der mindestens eine Beleuchtungspfad zwischen der Lichtquelle und der Metallgitterstruktur ein polarisationsmischendes Medium auf. Dies kann beispielsweise eine strahlformende Linse oder ein Streukörper sein. Vorzugsweise kann das polarisationsmischende Medium als ein distalseitiger Teil der Lichtquelle ausgebildet sein. Im Falle einer beispielsweise weiß leuchtenden LED als Lichtquelle emittiert der Dioden-Chip ursprünglich blaues Licht, das durch eine distalseitige Phosphorschicht tritt, welche breitbandig gelbes Licht emittiert, sodass weißes Licht erzeugt wird. Die Phosphorschicht ist dabei ein Streukörper, der als polarisationsmischendes Medium zwischen Lichtquelle und Metallgitterstruktur dienen kann.

Optional kann der mindestens eine Beleuchtungspfad zwischen der Lichtquelle und der Metallgitterstruktur eine lichtstrahlformende Linse aufweisen. Diese kann das Beleuchtungsfeld wie gewünscht formen, beispielsweise mit einer breiten Auffächerung des Beleuchtungsfeldes oder mit einer Fokussierung des Lichts auf einen Beleuchtungsspot. Die Linse kann zudem als polarisationsmischendes Medium dienen.

Optional kann die Metallgitterstruktur distalwärtig durch ein Abschlussfenster und/oder distalseitig durch eine Schutzschicht geschützt sein. Dies ist vorteilhaft, damit die Metallgitterstruktur nicht verkratzt oder sonstigen äußeren Einflüssen direkt ausgesetzt ist. Das Abschlussfenster bzw. die Schutzschicht sind dabei nicht lichtstrahlformend, stellen also keine Linse dar, um den Polarisationsgrad nicht zu beeinträchtigen. Die Metallgitterstruktur kann dabei proximalseitig auf dem Abschlussfenster aufgebracht sein. Alternativ dazu kann die Metallgitterstruktur distalseitig auf dem Abschlussfenster aufgebracht sein und ihrerseits distalseitig mit einer Schutzschicht bedeckt sein. Das dabei proximal von der Metallgitterstruktur angeordnete Abschlussfenster kann distalseitig planar und proximalseitig konkav sein, also selbst als strahlaufweitende Streulinse zwischen der Lichtquelle und der Metallgitterstruktur dienen. Die Schutzschicht kann jeweils relativ dünn, hart, kratzfest sowie chemisch und thermisch resistent sein, z.B. eine SiO₂-Schicht bzw. Quarzglasschicht.

Optional kann die Metallgitterstruktur distalseitig auf der Lichtquelle, distalseitig auf einer lichtstrahlformenden Linse oder auf einem Abschlussfenster fotolithographisch aufgedampft sein.

In einer bevorzugten Ausführungsform kann der mindestens eine Beleuchtungspfad ein erster von mindestens zwei Beleuchtungspfaden des Einführabschnitts sein und der Einführabschnitt einen zweiten der mindestens zwei Beleuchtungspfade aufweisen, wobei der zweite Beleuchtungspfad dazu eingerichtet ist, im Wesentlichen unpolarisiertes Licht zu emittieren, wobei das Instrument wahlweise in einem ersten Betriebsmodus mit Beleuchtung mittels zumindest teilweise polarisierten Lichts über den ersten Beleuchtungspfad und in einem zweiten Betriebsmodus mit Beleuchtung mittels im Wesentlichen unpolarisierten Lichts über den zweiten Beleuchtungspfad betreibbar ist, wobei das Instrument zwischen dem ersten und zweiten Betriebsmodus umschaltbar ist. Damit ermöglicht das Instrument ein wahlweises Umschalten zwischen Beleuchtung mit polarisiertem Licht, beispielsweise wenn gerade Reflexionen stören, und Beleuchtung mit unpolarisiertem Licht, beispielsweise wenn Reflexionen gerade nicht stören und ggf. mehr Licht benötigt wird. Da die Reflexionen stark vom Blickwinkel und der momentanen Position des beweglichen Instruments abhängen, kann damit die endoskopische Untersuchung verbessert werden.

In einer nicht beanspruchten Ausführungsform der Erfindung kann die Lichtquelle eine Auskopplung einer Lichtleitereinzelfaser des mindestens einen Beleuchtungspfads sein und das Instrument proximalseitig vom Einführabschnitt eine Laserlichteinkopplung in die Lichtleitereinzelfaser aufweisen, wobei zwischen der Auskopplung und der Metallgitterstruktur im Beleuchtungspfad eine laserstrahlaufweitende Linse angeordnet ist. Dies hat den Vorteil, dass Laserlicht bereits einen hohen Polarisationsgrad aufweisen kann und somit eine besonders hohe polarisierte Lichtausbeute ermöglicht wird. Ein oder mehrere Laserstrahlen können sehr stark kollimiert als kohärentes Lichtstrahlbündel in eine Lichtleitereinzelfaser eingekoppelt werden. Da eine Lichtleitereinzelfaser sehr dünn ausgestaltet werden kann, ist dies insbesondere bei dünnsten minimalinvasiven endoskopischen Instrumenten vorteilhaft. Da die laserstrahlaufweitende Linse für die flächige Beleuchtung den Polarisationsgrad beeinträchtigt, erhöht die Metallgitterstruktur den Polarisationsgrad wieder distalwärtig der Linse.

Optional kann bei der nicht beanspruchten Ausführungsform der Erfindung die Laserlichteinkopplung dazu ausgestaltet sein, im Wesentlichen polarisiertes Laserlicht eines Lasers und/oder polarisiertes Laserlicht als Mischung aus mehreren verschiedenfarbigen Lasern einzukoppeln.

Optional kann der Bildgebungspfad ein bildgebendes Element und distalwärts vom bildgebenden Element ein polarisierendes Medium aufweisen, wobei die Metallgitterstruktur in dem mindestens einen Beleuchtungspfad eine erste Polarisationsebene definiert und das polarisierende Medium im Bildgebungspfad eine zweite Polarisationsebene definiert, wobei sich die erste Polarisationsebene und die zweite Polarisationsebene kreuzen, d.h. im Wesentlichen entlang einer Längsachse des Einführabschnitts schneiden und einen festen Winkel von vorzugsweise etwa 90° einschließen.

Optional kann das polarisierende Medium im Bildgebungspfad ein dichroitischer Polarisator, eine Metallgitterstruktur und/oder ein doppelbrechendes Prisma sein.

Die Offenbarung ist nachfolgend anhand von einem in den Zeichnungen dargestellten Ausführungsbeispiel näher erläutert. Es zeigen:
Fig. 1 einen schematischen Längsschnitt durch einen distalen Abschnitt eines Einführabschnitts gemäß einem ersten Ausführungsbeispiel des hierin offenbarten endoskopischen Instruments;
Fig. 2 einen schematischen Längsschnitt durch einen distalen Abschnitt eines Einführabschnitts gemäß einem zweiten Ausführungsbeispiel des hierin offenbarten endoskopischen Instruments;
Fig. 3 einen schematischen Längsschnitt durch einen distalen Abschnitt eines Einführabschnitts gemäß einem zweiten Ausführungsbeispiel des hierin offenbarten endoskopischen Instruments; und
Fig. 4 eine schematische Frontansicht auf ein distales Ende eines Einführabschnitts gemäß einem Ausführungsbeispiel des hierin offenbarten endoskopischen Instruments.

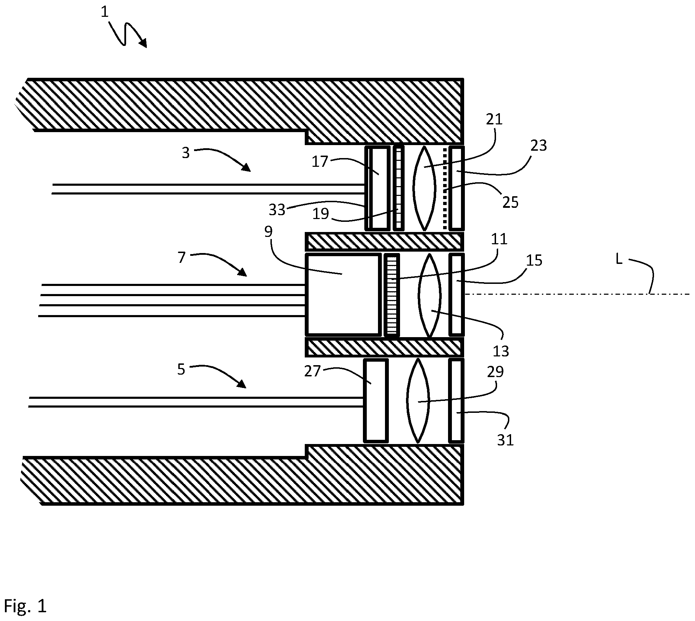

Fig. 1 zeigt einen distalen Endabschnitt eines Einführabschnitts 1 eines endoskopischen Instruments, wobei der Einführabschnitt 1 in einen Hohlraum eines tierischen oder menschlichen Körpers zu diagnostischen und/oder therapeutischen Zwecken minimalinvasiv einführbar ist. Alternativ dazu kann der Einführabschnitt 1 in einen Hohlraum eines technischen Geräts einführbar sein, um dieses untersuchen und/oder reparieren zu können. Der hier gezeigte Einführabschnitt 1 weist dazu einen ersten Beleuchtungspfad 3 und einen zweiten Beleuchtungspfad 5 auf, wobei der zweite Beleuchtungspfad 5 optional ist. Der erste Beleuchtungspfad 3 dient der Beleuchtung des Hohlraums mit zumindest teilweise polarisiertem Licht. Der zweite Beleuchtungspfad 5 kann der Beleuchtung des Hohlraums mit unpolarisiertem Licht dienen. Falls der Einführabschnitt 1 einen zweiten Beleuchtungspfad 5, wie in Figuren 1 bis 4 gezeigt, aufweist, ist das endoskopische Instrument vorzugsweise umschaltbar zwischen einem ersten Betriebsmodus mit Beleuchtung mittels zumindest teilweise polarisierten Lichts über den ersten Beleuchtungspfad 3 und einem zweiten Betriebsmodus mit Beleuchtung mittels unpolarisierten Lichts über den zweiten Beleuchtungspfad 5.

Zudem weist der Einführabschnitt 1 hier zentral und koaxial mit einer Längsachse L des Einführabschnitts 1 angeordnet einen Bildgebungspfad 7 auf. Der Bildgebungspfad 7 dient dazu, ein Bild vom beleuchteten Hohlraum aufzunehmen und entsprechende Signale proximalwärts zu leiten, um ein entsprechendes Bild vom beleuchteten Hohlraum beispielsweise auf einem externen Bildschirm darstellen zu können. Der Bildgebungspfad 7 weist an seinem distalen Ende ein bildgebendes Element in Form eines Bildsensors 9 auf, der beispielsweise ein CMOS-Sensor oder ein CCD-Sensor sein kann. Distalwärts vom Bildsensor 9 ist hier ein polarisierendes Medium in Form eines dichroitischen Linearpolarisationsfilters 11 angeordnet, damit der Bildsensor 9 im Wesentlichen nur solches Licht auf den Bildsensor 9 trifft, das in einer bestimmten Polarisationsebene polarisiert ist. Alternativ oder zusätzlich zum Linearpolarisationsfilter 11 kann als polarisierendes Medium eine Metallgitterstruktur und/oder ein doppelbrechendes Prisma zum Einsatz kommen. Distalwärts vom Bildsensor 9 ist außerdem ein Objektiv 13 angeordnet, das wiederum distalwärts mittels eines Abschlussfensters 15 gegen äußere Einflüsse geschützt ist.

Der erste Beleuchtungspfad 3 ist lateral vom zentralen Bildgebungspfad 7 angeordnet und weist an seinem distalen Ende eine Lichtquelle in Form einer LED 17 oder Laserdiode auf. Distalwärtig von der LED 17 ist ein polarisationsmischendes Medium 19 angeordnet, das als distalseitiger Teil der LED 17 ausgebildet sein kann. Zum Beispiel kann die LED 17 eine weiß leuchtende LED sein, deren Dioden-Chip ursprünglich blaues Licht emittiert, das durch eine distalseitige Phosphorschicht tritt, welche breitbandig gelbes Licht emittiert, sodass weißes Licht distalseitig aus der Phosphorschicht austritt. Die Phosphorschicht ist dabei ein Streukörper, der als polarisationsmischendes Medium 19 dienen kann.

Der erste Beleuchtungspfad 3 weist zudem distalwärts vom polarisationsmischenden Medium 19 eine strahlformende Linse 21 auf, die hier als Sammellinse dargestellt ist, allerdings je nach Bedarf auch als Streulinse oder anders geformt sein kann. Mit der Linse 21 kann der auszuleuchtende Bereich des Hohlraums bestimmt werden, beispielsweise als relativ fokussierter Spot oder als eher breit aufgeweitetes Beleuchtungsfeld. Distalwärts wird die Linse 21 analog zum Objektiv von einem Abschlussfenster 23 gegen äußere Einflüsse geschützt. Proximalseitig ist auf das Abschlussfenster23 eine Metallgitterstruktur25 fotolithographisch aufgedampft.

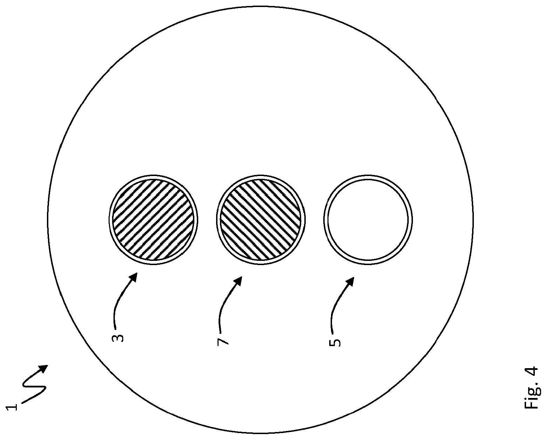

Der erste Beleuchtungspfad 3 weist keinen Linearpolarisationsfilter wie der Bildgebungspfad 7 auf. Die Metallgitterstruktur 25 transmittiert einen ersten Anteil des Lichts der Lichtquelle mit einer bestimmten linearen Polarisation zumindest teilweise distalwärts und reflektiert einen zweiten Anteil des Lichts der Lichtquelle ohne diese Polarisation zumindest teilweise proximalwärts. Die Polarisationsebenen der Metallgitterstruktur 25 im ersten Beleuchtungspfad 3 und des Linearpolarisationsfilters 11 im Bildgebungspfad 7 sind vorzugsweise 90° zueinander gekreuzt, wie in Fig. 4 schematisch verdeutlicht. Das von der Metallgitterstruktur 25 transmittierte linear polarisierte Licht, das an einer spiegelnden Oberfläche des Hohlraums, wie etwa Schleim, polarisationserhaltend reflektiert wird, wird damit vom Linearpolarisationsfilter 11 absorbiert und trifft nicht auf dem Bildsensor 9. Das von der Metallgitterstruktur 25 transmittierte linear polarisierte Licht, das an der Struktur des Hohlraums, wie etwa der Schleimhaut, polarisationsmischend gestreut wird, wird hingegen zumindest teilweise vom Linearpolarisationsfilter 11 durchgelassen und trifft auf dem Bildsensor 9. Damit ist die Bildgebung mit Beleuchtung über den ersten Beleuchtungspfad 3 erheblich reflexreduziert.

Falls eine reflexreduzierte Bildgebung in dem Maße nicht benötigt wird, kann ein Benutzer auf eine Beleuchtung über den zweiten Beleuchtungspfad 5 umschalten und den Hohlraum mit unpolarisiertem Licht ausleuchten. Dabei wird der Linearpolarisationsfilter 11 ggf. solche Lichtanteile zum Bildsensor 9 durchlassen, die in der Polarisationsebene des Linearpolarisationsfilter 11 liegen und polarisationserhaltend von spiegelnden Oberflächen des Hohlraums reflektiert wurden. Der zweite Beleuchtungspfad 5 kann eine andere Lichtquelle oder die gleiche Lichtquelle wie der erste Beleuchtungspfad 3 aufweisen. Vorzugsweise ist dort wie im ersten Beleuchtungspfad 3 eine LED 27, eine Linse 29 und ein Abschlussfenster 31 angeordnet. Es fehlt im zweiten Beleuchtungspfad 5 lediglich die Metallgitterstruktur 25. Für den Fall, dass die LED 27 im zweiten Beleuchtungspfad 5 nicht selbst bereits ein polarisationsmischendes Medium 19 aufweist, weist der zweite Beleuchtungspfad 5 dieses vorzugsweise auch nicht auf.

Die reflexreduzierte Bildgebung mit Beleuchtung über den ersten Beleuchtungspfad 3 ist besonders allerdings effizient, da sich die Metallgitterstruktur 25 im Gegensatz zum einem absorbierenden Linearpolarisationsfilter nicht so stark aufheizt, weil sie den zweiten Anteil des Lichts der LED 17, dem die transmittierte Polarisation fehlt, proximalwärts zur LED 17 zurückreflektiert und diesen nicht absorbiert. Dies hat den weiteren Vorteil, dass dieser zweite Anteil des Lichts nicht vollständig verloren ist, sondern teilweise "recycelt" wird. Zwar ist der von der Metallgitterstruktur 25 zurückreflektierte zweite Anteil des Lichts der LED 17, dem die transmittierte Polarisation fehlt, zunächst selbst komplementär polarisiert, allerdings hebt das polarisationsmischende Medium 19 diese komplementäre Polarisation wieder auf. Da die LED 17 proximalseitig eine distalwärts reflektierende Schicht 33 aufweist, wird der dann polarisationsgemischte zweite Lichtanteil wieder dem neu erzeugten Licht der LED 17 hinzugefügt und abermals distalwärts von der LED 17 emittiert. Da dieses "Recycling" mehrfach durchlaufen wird, ist die Lichtausbeute von polarisiertem Licht, das letztlich von der Metallgitterstruktur 25 distalwärts transmittiert wird, erheblich höher als wenn ein absorbierender Linearpolarisationsfilter im ersten Bildgebungspfad 3 verwendet würde.

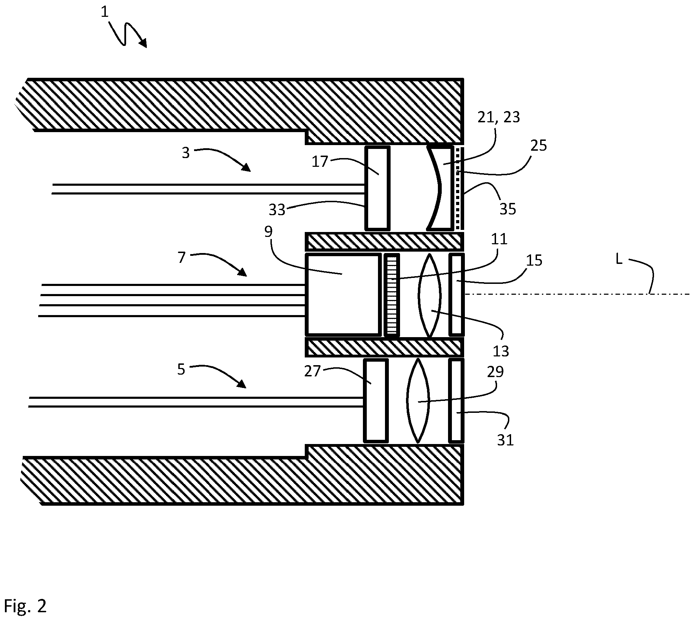

Im Gegensatz zu dem in Fig. 1 dargestellten Ausführungsbeispiel wird in dem in Fig. 2 gezeigten Ausführungsbeispiel anstatt einer Sammellinse eine Streulinse als strahlformende Linse 21 im ersten Bildgebungspfad 3 verwendet, um ein breit aufgefächertes Beleuchtungsfeld zu erzielen. Die Streulinse 21 ist dabei proximalseitig konkav und distalseitig planar ausgebildet. Hier ist die Metallgitterstruktur 25 auf der planaren Distalseite der Streulinse 21 fotolithografisch aufgedampft. Die Metallgitterstruktur 25 ist ihrerseits distalseitig durch eine Schutzschicht 35 aus SiO₂ bzw. Quarzglas gegen äußere Einflüsse geschützt. Es könnte auch ein zusätzliches distales Abschlussfenster 23 wie in Fig. 1 verwendet werden, beispielsweise im Falle einer distalseitig konkav ausgebildeten Streulinse, wobei die Metallgitterstruktur 25 proximalseitig oder distalseitig auf dem Abschlussfenster 23 fotolithografisch aufgedampft und, falls distalseitig aufgedampft, ihrerseits distalseitig durch eine Schutzschicht 35 aus SiO₂ bzw. Quarzglas gegen äußere Einflüsse geschützt sein kann. In Fig. 2 bildet die Streulinse 21 selbst das Abschlussfenster 23 und wirkt hier zumindest teilweise polarisationsmischend, sodass es hier keines zusätzlichen polarisationsmischenden Mediums 19 bedarf. Allerdings kann die LED 17 wie zuvor distalseitig ein polarisationsmischendes Medium 19 aufweisen.

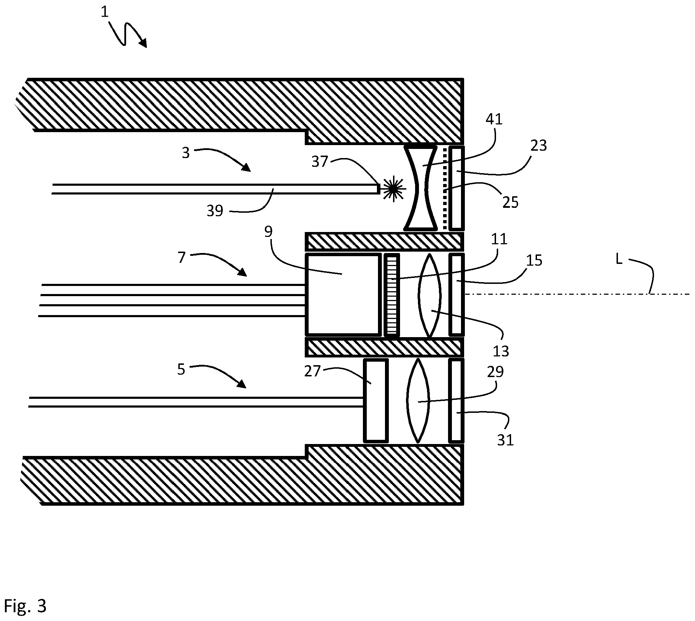

In Fig. 3 kommt am distalen Ende des ersten Beleuchtungspfads 3 keine LED als Lichtquelle zum Einsatz, sondern eine Auskopplung 37 einer Lichtleitereinzelfaser 39, in die proximalseitig vom Einführabschnitt 1 Laserlicht über eine Laserlichteinkopplung eingekoppelt wird. Zwischen der Auskopplung 37 und der Metallgitterstruktur 25, die hier proximalseitig auf dem distalen Abschlussfenster23 aufgedampft ist, ist eine beidseitig konkav ausgebildete Streulinse 41 als laserstrahlaufweitende Linse angeordnet. Das Laserlicht weist vorzugsweise schon einen relativ hohen Polarisationsgrad auf, allerdings wird dieser durch den polarisationsmischenden Effekt der Streulinse 41 geschwächt. Die Metallgitterstruktur 25 kompensiert diese Schwächung des Polarisationsgrads wieder, sodass auf effiziente Weise ein breit ausgefächertes Beleuchtungsfeld mit polarisiertem Laserlicht erzeugt werden kann. Dazu kann polarisiertes Laserlicht eines Lasers und/oder polarisiertes Laserlicht als Mischung aus mehreren verschiedenfarbigen Lasern in die Lichtleitereinzelfaser 39 eingekoppelt werden.

Die nummerierten Bezeichnungen der Bauteile oder Bewegungsrichtungen als "erste", "zweite ... "dritte" usw. sind hierin rein willkürlich zur Unterscheidung der Bauteile oder Bewegungsrichtungen untereinander gewählt und können beliebig anders gewählt werden. Es ist damit kein Bedeutungsrang verbunden. Eine Bezeichnung eines Bauteils oder technischen Merkmals als "erstes" soll nicht dahingehend missverstanden werden, dass es ein zweites Bauteil oder technisches Merkmal dieser Art geben muss. Außerdem können etwaige Verfahrensschritte, soweit nicht explizit anders erläutert oder zwingend erforderlich, in beliebiger Reihenfolge und/oder zeitlich teilweise oder ganz überlappend durchgeführt werden.

Als optional, vorteilhaft, bevorzugt, erwünscht oder ähnlich bezeichnete "kann"-Merkmale sind als optional zu verstehen und nicht als schutzbereichsbeschränkend.

Die beschriebenen Ausführungsformen sind als illustrative Beispiele zu verstehen und stellen keine abschließende Liste von möglichen Ausführungsformen dar. Jedes Merkmal, das im Rahmen einer Ausführungsform offenbart wurde, kann allein oder in Kombination mit einem oder mehreren anderen Merkmalen verwendet werden, unabhängig davon, in welcher Ausführungsform die Merkmale jeweils beschrieben wurden. Während mindestens ein Ausführungsbeispiel hierin beschrieben und gezeigt ist, seien Abwandlungen und alternative Ausführungsformen, die einer fachmännisch versierten Person in Anbetracht dieser Beschreibung als offensichtlich erscheinen, vom Schutzbereich dieser Offenbarung mit erfasst. Im Übrigen soll hierin weder der Begriff "aufweisen" zusätzliche andere Merkmale oder Verfahrensschritte ausschließen noch soll "ein" oder "eine" eine Mehrzahl ausschließen.

### Bezugszeichenliste:

- 1: Einführabschnitt eines endoskopischen Instruments
- 3: erster Beleuchtungspfad
- 5: zweiter Beleuchtungspfad
- 7: Bildgebungspfad
- 9: bildgebendes Element
- 11: Linearpolarisationsfilter
- 13: Objektiv
- 15: Abschlussfensterim Bildgebungspfad
- 17: LED im ersten Beleuchtungspfad
- 19: polarisationsmischendes Medium
- 21: lichtstrahlformende Linse im ersten Beleuchtungspfad
- 23: Abschlussfenster im ersten Beleuchtungspfad
- 25: Metallgitterstruktur
- 27: LED im zweiten Beleuchtungspfad
- 29: lichtstrahlformende Linse im zweiten Beleuchtungspfad
- 31: Abschlussfenster im zweiten Beleuchtungspfad
- 33: distalwärts reflektierende Schicht
- 35: Schutzschicht
- 37: Lichtleitereinzelfaserauskopplung
- 39: Lichtleitereinzelfaser
- 41: laserstrahlaufweitende Linse
- L: Längsachse des Einführabschnitts

## Patentansprüche

1. Endoskopisches Instrument mit einem distalen länglichen Einführabschnitt (1) zum Einführen in einen Hohlraum, wobei der Einführabschnitt (1) mindestens einen Beleuchtungspfad (3) und einen Bildgebungspfad (7) aufweist, wobei der mindestens eine Beleuchtungspfad (3) an einem distalen Ende des Einführabschnitts (1) eine LED (17) aufweist,
**dadurch gekennzeichnet, dass**
der mindestens eine Beleuchtungspfad (3) distalwärtig von der LED (17) eine Metallgitterstruktur (25) aufweist, die einen ersten Anteil des Lichts der LED (17) mit einer bestimmten linearen Polarisation zumindest teilweise distalwärts transmittiert und einen zweiten Anteil des Lichts der LED (17) ohne diese Polarisation zumindest teilweise proximalwärts reflektiert, und
der mindestens eine Beleuchtungspfad (3) distalwärtig von der Metallgitterstruktur (25) linsenfrei ist, wobei die LED (17) proximalseitig eine reflektierende Schicht (33) aufweist, die zumindest teilweise den von der Metallgitterstruktur (25) proximalwärts reflektierten zweiten Lichtanteil distalwärts reflektiert, wobei der mindestens eine Beleuchtungspfad (3) zwischen der LED (17) und der Metallgitterstruktur (25) ein polarisationsmischendes Medium (19) aufweist.

2. Endoskopisches Instrument nach Anspruch 1, wobei das polarisationsmischende Medium (19) als ein distalseitiger Teil der LED (17) ausgebildet ist.

3. Endoskopisches Instrument nach einem der vorhergehenden Ansprüche, wobei der mindestens eine Beleuchtungspfad (3) zwischen der LED (17) und der Metallgitterstruktur (25) eine lichtstrahlformende Linse (21) aufweist.

4. Endoskopisches Instrument nach einem der vorhergehenden Ansprüche, wobei die Metallgitterstruktur (25) distalwärtig durch ein Abschlussfenster (23) und/oder distalseitig durch eine Schutzschicht (35) geschützt ist.

5. Endoskopisches Instrument nach einem der vorhergehenden Ansprüche, wobei der mindestens eine Beleuchtungspfad (3) ein distales Abschlussfenster (23) aufweist, wobei die Metallgitterstruktur (25) distalseitig auf dem Abschlussfenster (23) aufgebracht und mit einer Schutzschicht (35) bedeckt ist.

6. Endoskopisches Instrument nach einem der vorhergehenden Ansprüche, wobei der mindestens eine Beleuchtungspfad (3) ein distales Abschlussfenster (23) aufweist, wobei die Metallgitterstruktur (25) proximalseitig auf dem Abschlussfenster (23) aufgebracht ist.

7. Endoskopisches Instrument nach einem der vorhergehenden Ansprüche, wobei die Metallgitterstruktur (25) distalseitig auf der LED (17), distalseitig auf einer lichtstrahlformenden Linse (21) oder auf einem Abschlussfenster (23) fotolithographisch aufgedampft ist.

8. Endoskopisches Instrument nach einem der vorhergehenden Ansprüche, wobei der mindestens eine Beleuchtungspfad (3) ein proximal von der Metallgitterstruktur angeordnetes Abschlussfenster (23) aufweist, wobei das Abschlussfenster (23) eine strahlaufweitende Streulinse (21) bildet und distalseitig planar und proximalseitig konkav ist.

9. Endoskopisches Instrument nach einem der vorhergehenden Ansprüche, wobei der mindestens eine Beleuchtungspfad (3) ein erster (3) von mindestens zwei Beleuchtungspfaden (3, 5) des Einführabschnitts (1) ist und der Einführabschnitt (1) einen zweiten (5) der mindestens zwei Beleuchtungspfade (3, 5) aufweist, wobei der zweite Beleuchtungspfad (5) dazu eingerichtet ist, im Wesentlichen unpolarisiertes Licht zu emittieren, wobei das Instrument wahlweise in einem ersten Betriebsmodus mit Beleuchtung mittels zumindest teilweise polarisierten Lichts überden ersten Beleuchtungspfad (3) und in einem zweiten Betriebsmodus mit Beleuchtung mittels im Wesentlichen unpolarisierten Lichts über den zweiten Beleuchtungspfad (5) betreibbar ist, wobei das Instrument zwischen dem ersten und zweiten Betriebsmodus umschaltbar ist.

10. Endoskopisches Instrument nach einem der vorhergehenden Ansprüche, wobei der Bildgebungspfad (7) ein bildgebendes Element (9) und distalwärts vom bildgebenden Element (9) ein polarisierendes Medium (11) aufweist, wobei die Metallgitterstruktur (25) in dem mindestens einen Beleuchtungspfad (3) eine erste Polarisationsebene definiert und das polarisierende Medium (11) im Bildgebungspfad (7) eine zweite Polarisationsebene definiert, wobei sich die erste Polarisationsebene und die zweite Polarisationsebene kreuzen.

11. Endoskopisches Instrument nach Anspruch 10, wobei das polarisierende Medium (11) im Bildgebungspfad (7) ein dichroitischer Polarisator, eine Metallgitterstruktur und/oder ein doppelbrechendes Prisma ist.

## Claims

1. An endoscopic instrument having a distal elongate insertion portion (1) for insertion into a cavity, wherein the insertion portion (1) has at least one illumination path (3) and an imaging path (7), wherein the at least one illumination path (3) has an LED (17) at a distal end of the insertion portion (1),
**characterised in that**
the at least one illumination path (3) has, distally of the LED (17), a metal lattice structure (25), which transmits a first component of the light of the LED (17) with a certain linear polarisation at least partially distally and reflects a second component of the light of the LED (17) without this polarisation at least partially proximally, and
the at least one illumination path (3) is lens-free distally of the metal lattice structure (25), wherein the LED (17) has a reflective layer (33) proximally, which at least partially reflects distally the second light component reflected proximally by the metal lattice structure (25), wherein the at least one illumination path (3) has a polarisation-mixing medium (19) between the LED (17) and the metal lattice structure (25).

2. The endoscopic instrument according to claim 1, wherein the polarisation-mixing medium (19) is formed as a distal-side part of the LED (17).

3. The endoscopic instrument according to one of the preceding claims, wherein the at least one illumination path (3) has a light-beam-shaping lens (21) between the LED (17) and the metal lattice structure (25).

4. The endoscopic instrument according to one of the preceding claims, wherein the metal lattice structure (25) is protected distally by a termination window (23) and/or on the distal side by a protective layer (35).

5. The endoscopic instrument according to one of the preceding claims, wherein the at least one illumination path (3) has a distal termination window (23), wherein the metal lattice structure (25) is applied distally to the termination window (23) and is covered by a protective layer (35).

6. The endoscopic instrument according to one of the preceding claims, wherein the at least one illumination path (3) has a distal termination window (23), wherein the metal lattice structure (25) is applied proximally to the termination window (23).

7. The endoscopic instrument according to one of the preceding claims, wherein the metal lattice structure (25) is applied photolithographically by vapour deposition to the distal side of the LED (17), to the distal side of a light-beam-shaping lens (21) or to a termination window (23).

8. The endoscopic instrument according to one of the preceding claims, wherein the at least one illumination path (3) has a termination window (23) arranged proximally of the metal lattice structure, wherein the termination window (23) forms a beam-widening diffuser lens (21) and is planar on the distal side and concave on the proximal side.

9. The endoscopic instrument according to one of the preceding claims, wherein the at least one illumination path (3) is a first (3) of at least two illumination paths (3, 5) of the insertion portion (1) and the insertion portion (1) has a second (5) of the at least two illumination paths (3, 5), wherein the second illumination path (5) is designed to emit substantially unpolarised light, wherein the instrument is operable selectively in a first operating mode with illumination by means of at least partially polarised light via the first illumination path (3) and in a second operating mode with illumination by means of substantially unpolarised light via the second illumination path (5), wherein the instrument is switchable between the first and second operating mode.

10. The endoscopic instrument according to one of the preceding claims, wherein the imaging path (7) has an imaging element (9) and distally of the imaging element (9) a polarising medium (11), wherein the metal lattice structure (25) defines a first polarisation plane in the at least one illumination path (3) and the polarising medium (11) defines a second polarisation plane in the imaging path (7), wherein the first polarisation plane and the second polarisation plane cross one another.

11. The endoscopic instrument according to claim 10, wherein the polarising medium (11) in the imaging path (7) is a dichroitic polariser, a metal lattice structure and/or a birefringent prism.

## Revendications

1. Instrument endoscopique avec une partie d'introduction (1) allongée distale destinée à l'introduction dans un espace creux, la partie d'introduction (1) comprenant au moins un chemin d'éclairage (3) et un chemin d'imagerie (7), ledit au moins un chemin d'éclairage (3) comportant, à une extrémité distale de la partie d'introduction (1), une DEL (17),
**caractérisé en ce que**
ledit au moins un chemin d'éclairage (3) comprend, du côté distal par rapport à la DEL (17), une structure de grille métallique (25) qui transmet une première partie de la lumière de la DEL (17), avec une polarisation linéaire déterminée, au moins partiellement vers le côté distal et qui réfléchit une deuxième partie de la lumière de la DEL (17), sans cette polarisation, au moins partiellement vers le côté proximal et
ledit au moins un chemin d'éclairage (3) est sans lentille vers le côté distal par rapport à la structure de grille métallique (25), la DEL (17) comprenant du côté proximal une couche réfléchissante (33) qui réfléchit la deuxième partie de lumière réfléchie par la structure de grille métallique (25) vers le côté proximal, au moins partiellement vers le côté distal, ledit au moins un chemin d'éclairage (3) comprenant un medium mélangeur de polarisation (19) entre la DEL (17) et la structure de grille métallique (25).

2. Instrument endoscopique selon la revendication 1, dans lequel le médium mélangeur de polarisation (19) a la forme d'une partie de côté distal de la DEL (17).

3. Instrument endoscopique selon l'une des revendications précédentes, dans lequel ledit au moins un chemin d'éclairage (3) comprend une lentille (21) formant un rayon lumineux, entre la DEL (17) et la structure de grille métallique (25).

4. Instrument endoscopique selon l'une des revendications précédentes, dans lequel la structure de grille métallique (25) est protégée vers le côté distal par une fenêtre de fermeture (23) et/ou du côté distal par une couche de protection.

5. Instrument endoscopique selon l'une des revendications précédentes, dans lequel ledit au moins chemin d'éclairage (3) comprend une fenêtre de fermeture distale (23), la structure de grille métallique (25) étant déposée du côté distal sur la fenêtre de fermeture (25) et étant recouverte d'une couche de protection (35).

6. Instrument endoscopique selon l'une des revendications précédentes, dans lequel ledit au moins chemin d'éclairage (3) comprend une fenêtre de fermeture (23) distale, la structure de grille métallique (25) étant déposée du côté proximal sur la fenêtre de fermeture (23).

7. Instrument endoscopique selon l'une des revendications précédentes, dans lequel la structure de grille métallique (25) est déposée en phase vapeur de manière photolithographique du côté distal sur la DEL (17), du côté distal sur une lentille (21) formant un rayon lumineux ou sur une fenêtre de fermeture (23).

8. Instrument endoscopique selon l'une des revendications précédentes, dans lequel ledit au moins chemin d'éclairage (3) comprend une fenêtre de fermeture (23) disposée du côté proximal par rapport à la structure de grille métallique, la fenêtre de fermeture (23) formant une lentille divergente et étant plane du côté distal et concave du côté proximal.

9. Instrument endoscopique selon l'une des revendications précédentes, dans lequel ledit au moins un chemin d'éclairage (3) est un premier (3) d'au moins deux chemins d'éclairage (3, 5) de la partie d'introduction (1), et la partie d'introduction (1) comprend un deuxième (5) desdits au moins deux chemins d'éclairage (3, 5), le deuxième chemin d'éclairage (5) étant configuré pour émettre de la lumière essentiellement non polarisée, l'instrument pouvant être actionné au choix dans un premier mode de fonctionnement avec un éclairage au moyen d'une lumière au moins partiellement polarisée passant par le premier chemin d'éclairage (3), et dans un deuxième mode de fonctionnement avec un éclairage au moyen d'une lumière essentiellement non polarisée passant par le deuxième chemin d'éclairage (5), l'instrument pouvant être commuté entre le premier et le deuxième mode de fonctionnement.

10. Instrument endoscopique selon l'une des revendications précédentes, dans lequel le chemin d'imagerie (7) comprend un élément d'imagerie (9) et, vers le côté distal par rapport à l'élément d'imagerie (9), un médium polarisant (11), la structure de grille métallique (25) définissant dans ledit au moins un chemin d'éclairage (3) un premier plan de polarisation et le médium polarisant (11) définissant dans le chemin d'imagerie (7) un deuxième plan de polarisation, le premier plan de polarisation et le deuxième plan de polarisation se croisant.

11. Instrument endoscopique selon la revendication 10, dans lequel le médium polarisant (11) dans le chemin d'imagerie (7) est un polariseur dichroïque, une structure de grille métallique et/ou un prisme biréfringent.
